(19) Europäisches Patentamt
European Patent Office
Office européen des brevets

(11) EP 1 303 626 B1

(12) EUROPEAN PATENT SPECIFICATION

(45) Date of publication and mention
of the grant of the patent:
**27.06.2007 Bulletin 2007/26**

(21) Application number: **01952007.1**

(22) Date of filing: **19.07.2001**

(51) Int Cl.:
***C12N 15/63*** (2006.01)

(86) International application number:
**PCT/KR2001/001231**

(87) International publication number:
**WO 2002/006493 (24.01.2002 Gazette 2002/04)**

(54) **RECOMBINANT PLASMID PSP1, MICROORGANISMS TRANSFORMED THEREWITH, AND METHOD FOR PRODUCING AN ALKALINE PROTEASE VAPK**

REKOMBINANTES PLASMID PSP1, MIT DIESEM TRANSFORMIERTE MIKROORGANISMEN UND VERFAHREN ZUR HERSTELLUNG EINER ALKALINEN VAPK-PROTEASE

PLASMIDE RECOMBINE PSP1, MICRO-ORGANISMES TRANSFORMES A L'AIDE DE CE PLASMIDE, ET PROCEDE DE PRODUCTION D'UNE PROTEASE ALCALINE VAPK

(84) Designated Contracting States:
**AT BE CH CY DE DK ES FI FR GB GR IE IT LI LU MC NL PT SE TR**

(30) Priority: **19.07.2000 KR 2000041213**

(43) Date of publication of application:
**23.04.2003 Bulletin 2003/17**

(73) Proprietor: **Cheil Jedang Corporation Seoul 100-095 (KR)**

(72) Inventors:
• **JIN, Ghee Hong Seoul 158-050 (KR)**
• **LEE, Hyune Hwan Dep. Bios. and Biotech. Hankuk Uni Gyeonggi-do 449-791 (KR)**
• **RHO, Hyune Mo 7th Floor, CJ Building Seoul 100-749 (KR)**
• **KIM, Hyung Seok Inchon 400-103 (KR)**

(74) Representative: **GROSSE BOCKHORNI SCHUMACHER Patent- und Rechtsanwälte Elsenheimerstrasse 49 80687 München (DE)**

(56) References cited:
**WO-A-00/61769      KR-A- 2000 065 867
US-A- 4 966 846**

• **SUN C S ET AL: "Transformation of an alkaline protease overproducer, Vibrio metschnikovii strain RH530, and improvement of plasmid stability by the par locus" JOURNAL OF MICROBIOLOGY AND BIOTECHNOLOGY 2001 SOUTH KOREA, vol. 11, no. 2, 2001, pages 222-228, XP009034100 ISSN: 1017-7825**
• **KIM J-Y ET AL: "Comparison of effects of the par locus and the parB locus on plasmid stability and host cell growth" JOURNAL OF FERMENTATION AND BIOENGINEERING 1996 JAPAN, vol. 82, no. 5, 1996, pages 495-497, XP002289230 ISSN: 0922-338X**

**Description**

TECHNICAL FIELD

The present invention relates to a recombinant plasmid vector pSP1 comprising the gene *vapk* encoding an alkaline protease VapK and the *par* gene increasing the stability of plasmid vector itself, a microorganism *Vibrio metschnikovii* transformed therewith, and a method for producing an alkaline protease VapK using the same microorganism.

BACKGROUND ART

In general, an alkaline protease derived from *Vibrio* sp. is referred to as an enzyme that degrades proteins into amino acids and small peptides, and many microorganisms, such as *Bacillus* sp. and *Serratia* sp., are well-known to excrete alkaline protease

The most important factor in producing alkaline protease using the said microorganisms is the high productivity of alkaline protease, which affects the costs of producing process.

According to the recent studies, recombinant vectors comprising the gene encoding alkaline protease were prepared using genetic engineering techniques and transformants therewith were also prepared in order to raise the productivity of alkaline protease. In addition, since the stability of the plasmid vector may directly affect the productivity of the alkaline protease, several studies related to the improvement of stability of recombinant plasmid vectors have been reported.

The present inventors previously filed a patent application (KR 1999-12588) titled "alkaline protease VapK suitable for laundry detergent, *vapk* gene, recombinant expression vector, and transformed microorganism", in which the microorganism is *Vibrio metschnikovii* KS 1 and the recombinant expression vector is pSBCm. The same microorganism and recombinant expression vector are also used in the present invention.

The present inventors have studied and tested intensively and extensively in order to produce the transformed microorganism with the recombinant plasmid vector with improved stability. After all, it was prepared a recombinant plasmid vector comprising the alkaline protease gene, isolated from *Vibrio metschnikovii* KS1 and the *par* gene with the function of increasing the stability of plasmid vector itself, and transformed *Vibrio metschnikovii* with the said recombinant plasmid vector, thereby the transformed microorganism comprising plasmid vector having improved stability as well as improved protease activity was also prepared.

SUMMARY OF THE INVENTION

The present invention provides a recombinant plasmid vector pSP1 in which the *par* gene is cloned into a recombinant plasmid vector pSBCm (KCCM-10142) comprising the *vapk* gene encoding an alkaline protease to improve the stability of plasmid vector itself.

The present invention also provides a method for producing the recombinant plasmid vector pSP1 of claim 1, by inserting the *par* gene into between *Pvu*II and *Hinc*II recognition sites of a recombinant plasmid vector pSBCm (KCCM-10142) comprising the *vapk* gene encoding an alkaline protease derived from *Vibrio* sp., wherein the *par* gene has the function of improving the stability of the plasmid vector itself.

Also, the present invention provides a transformed host cell comprising the recombinant plasmid vector pSP1.

Furthermore, the present invention provides a transformed host cell, where the host cell comprises *Escherichia coli* or *Vibrio metschnikovii.*

The present invention also provides a method for producing an alkaline protease VapK using the transformed host cell mentioned above.

BRIEF DESCRIPTION OF DRAWINGS

Hereinafter, the present invention is described in detail by referring to the attached drawings.

FIG. 1 illustrates the result of electrophoresis of the recombinant plasmid vector pSBCm comprising the vapk gene encoding an alkaline protease VapK. Line M represents size markers, line 1 represents a recombinant plasmid vector pSBCm, and line 2 represents a recombinant plasmid vector pSBCm/*Hin*d III consisting of a vehicle vector pKF3 of 2.2 kb and a *Vibrio* alkaline protease gene of 2.9 kb.

FIG. 2 illustrates the restriction enzyme map of the recombinant plasmid vector pSBCm comprising the alkaline protease gene *vapk.*

FIG. 3 illustrates the restriction enzyme map of the recombinant plasmid vector pSP 1 comprising the alkaline protease gene *vapk* and the *par* gene.

DETAILED DESCRIPTION OF THE INVENTION

The present invention provides a microorganism transformed with the recombinant plasmid vector comprising the gene *vapk* encoding an alkaline protease VapK and the *par* gene improving the stability of plasmid vector itself, and a method for improving the stability of the recombinant plasmid vector within the transformed microorganism mentioned above.

According to the present invention, the gene encoding the alkaline protease is isolated by the following steps: culturing *Vibrio metchnikovii* KS1, centrifuging and recovering the cells of *Vibrio metchnikovii* KS1, disrupturing the cells and centrifuging them to obtain the cell-free supernatant, and extracting the chromosomal DNA of *Vibrio metchnikovii* KS1 from the cell-free supernatant. Using the said chromosomal DNA of *Vibrio metchnikovii* KS1, the transformed *Escherichia coli* was produced, that is, by digesting the said chromosomal DNA of *Vibrio metchnikovii* KS1 with restriction enzymes like *Hin*d III to produce DNA fragment, cloning the DNA fragment into plasmid vectors to construct pSBCm and pSP1, and transforming them into *E. coli*.

First, the expression of the alkaline protease was conducted by using the above recombinant *E. coli* comprising plasmid vector pSBCm, and the expression level of the alkaline protease produced from the transformed *E. coli* under alkaline condition was measured. On the basis of this result, the expression of the alkaline protease from said transformed *E. coli* was low as known previously.

In order to solve the above problem, the present inventors cloned the recombinant plasmid vector pSBCm into *Vibrio* sp. As a result, however, the stability of the recombinant plasmid vector pSBCm was low. Therefore, in order to raise the stability of the said recombinant plasmid vector, the *par* gene that is known as being of the function of improving the stability of plasmid vector itself was inserted into the said recombinant plasmid vector pSBCm to construct the recombinant plasmid vector pSP1. As a result, the stability of the recombinant plasmid vector pSP1 was 40% higher than that of the recombinant plasmid vector pSBCm without the *par* gene.

The strain *Vibrio metchnikovii* KS1 that was deposited with the Korean Culture Center of Microorganisms, Seoul, Korea with an Accession Number of KFCC-10141 on December 15, 1998, used in the present invention, is produced by treating *Vibrio metchnikovii* RH 530 N-4-8 with N, N-nitrosoguanidine(NTG) as a mutagen, and that was deposited with the Korean Culture Center of Microorganisms with an Accession Number of KFCC-11030 on February 23, 1998. As a result, the amount of the alkaline protease produced from the *V. metschnikovii* KS 1 was twice as high as that of the strain *V. metschnikovii* RH 530 N-4-8.

Also, the strain *E. coli* Top10F' containing the recombinant plasmid vector pSBCm of the present invention was deposited with the Korean Culture Center of Microorganisms on December 15, 1998, as Accession No. KCCM-10142.

Hereinafter, a more detailed description on the specific effect of the present invention will be given by the following Examples, without limiting the spirit and scope of the invention.

EXAMPLES

Example 1

Construction of the recombinant plasmid vector pSBCm comprising the alkaline protease gene *vapk*

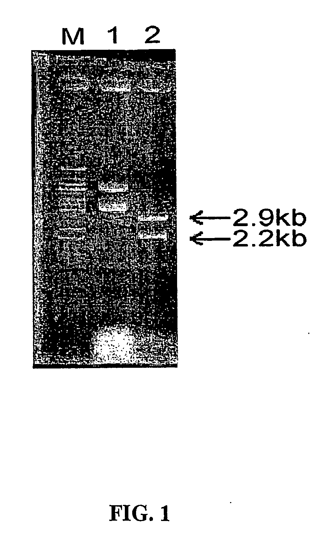

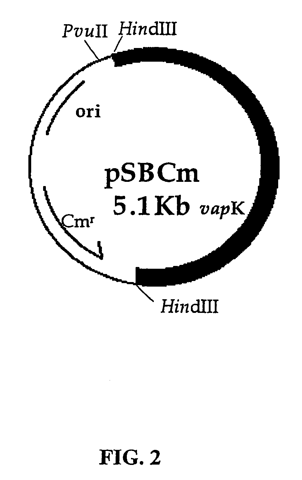

After culturing the *Vibrio metschnikovii* KS1 in a LSC medium with the composition of Table 1 at 30°C, the grown cells were harvested and disruptured. The disruptured solution was centrifuged at 6,000rpm and the supernatant was collected. The chromosomal DNA of *Vibrio metschnikovii* KS1 was purified from the supernatant and partially digested with Hind III. The fragment was inserted into a vector pKF3 to clone 2.9kb alkaline protease gene. The resulted recombinant plasmid vector was designated as pSBCm. The recombinant plasmid vector pSBCm was digested with Hind III and subjected to electrophoresis on 1% agarose gel. After electrophoresis, the agarose gel was stained with ethidium bromide as a staining agent. As a result of the above agarose gel electrophoresis, it was verified that the alkaline protease gene was cloned into a plasmid vector. (FIG. 1 and 2)

Table 1

| Composition of LSC medium | |
| --- | --- |
| Composition of LSC medium | Amount (g/L) |
| Tryptone | 10 |

(continued)

| Composition of LSC medium | |
|---|---|
| Composition of LSC medium | Amount (g/L) |
| Yeast extract | 5 |
| Sodium chloride | 10 |
| 1M Sodium carbonate buffer, pH 10.5 | 100(ml/L) |

Example 2

Construction of the recombinant plasmid vector pSP1 comprising the alkaline protease *vapk* gene and the *par* gene

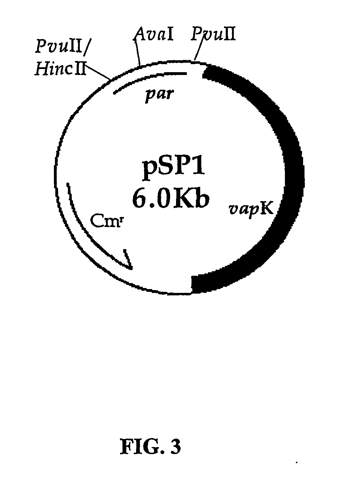

For the purpose of increasing the stability of the recombinant plasmid vector pSBCm, the *par* gene was cloned into a region between *Pvu*II and *Hinc*II recognition sites of pSBCm to prepare the recombinant plasmid vector pSP1 with the alkaline protease gene *vapk* and the *par* gene (FIG. 3).

Example 3

Transformation of *E. coli* with the recombinant plasmid vector comprising an alkaline protease gene

The recombinant plasmid vectors pSBCm and pSP1 comprising the 2.9kb alkaline protease gene of example 1 or example 2 was transformed into *E. coli* HB101.

After culturing the strain *E. coli* HB101 in a LB medium, 100mM calcium chloride was added into the culture broth of *E. coli* HB101. The mixture was stored at 0°C for 15 minutes and centrifuged to harvest *E. coli* HB101. The precipitated cells were suspended into a 100mM calcium chloride solution and stored at 0°C for 15 minutes. The recombinant plasmid vector pSBCm was added into the suspended solution and stored at 0°C for 1 hour. The suspended solution was heated at 42°C for 90 sec and stored at 0°C for 5 minutes. After adding 1ml of LB medium into the suspended solution, the solution was plated on LB agar medium containing 34 $\mu$g/ml of chloramphenicol and incubated at 30°C for 24 hours, and the colonies grown were selected.

Example 4

Transformation of *Vibrio metschnikovii* with the recombinant plasmid vector comprising the alkaline protease gene

An electroporation method was used for the transformation of *Vibrio metschnikovii* KS 1 with the recombinant plasmid vector comprising the alkaline protease gene.

After culturing the *Vibrio metschnikovii* KS1 in a LB medium at 30°C, the grown cells were harvested by centrifugation at 6,000rpm, 4°C for 10 minutes and suspended in a H-buffer solution consisting of 200mM sucrose, 1mM HEPES and 10% glycerol in the volume ratio of 20:1. After repeating the above process twice, and the cells were resuspended into a 80 $\mu\ell$ H-buffer solution. The recombinant plasmid vector was added to the suspended solution and the solution was poured into a 0.2cm cuvette. The electric power of 1,500V, 10$\mu$F and 200$\Omega$ was applied to the cuvette using Gene Pulser II made by Bio-Rad Co. The 600$\mu\ell$ of LB medium was added into the electric power-treated solution and incubated at 30°C. The cultured medium was plated on the LB agar medium containing 25$\mu$g/ml of chloramphenicol and the colonies grown were selected.

Example 5

Assay of the stability of the recombinant plasmid vector comprising the alkaline protease gene within the transformed *Vibrio* sp.

Stability of the recombinant plasmid vectors with or without the *par* gene was assayed and compared. Transformed *Vibrio metschnikovii* pSBCm and the transformed *Vibrio metschnikovii* pSP1 were individually seed-cultured in a LB medium containing 25 $\mu$g/ml of chloramphenicol for 18 hours and growth-cultured in a LB medium without chloramphenicol. During the growth culture, the cultured broth was sampled several times at a certain interval and each sample was immediately plated on each LB agar medium with or without 12.5$\mu$g/ml of chloramphenicol. After incubating the plated media at 30°C for 20 hours, the number of colonies was measured (Table 2). On the basis of the number of grown colonies, the stability of the recombinant plasmid vector may be calculated by the following equation:

$$\text{Stability of plasmid vector} = (A/B) \times 100$$

A: the number of colonies grown on LB agar medium containing 12.5 $\mu$g/ml of chloramphenicol; and
B: the number of colonies grown on LB agar medium without chloramphenicol.

Table 2

| The stability of the recombinant plasmid vector within the transformed strain *Vibrio metschnikovii* pSBCm and *Vibrio metschnikovii* pSP1 | | | | | |
|---|---|---|---|---|---|
| Time (hr)/ generation[a] | Growth (O.D. 600nm) | | | Stability of plasmid (%) | |
| | *Vibrio* sp. | pSBCm within *Vibrio* sp. | pSP1 within *Vibrio* sp | pSBCm within *Vibrio* sp. | pSP1 within *Vibrio* sp. |
| 6/4.5 | 2.41 | 2.16 | 2.25 | 51.72 | 64.83 |
| 12/18 | 4.27 | 3.66 | 3.32 | 37.72 | 62.38 |
| 18/27 | 5.31 | 4.00 | 3.42 | 24.56 | 41.99 |
| 27/40.5 | 5.98 | 4.41 | 4.04 | 22.00 | 37.07 |
| [a]One generation time is approximately 40 minutes. | | | | | |

INDUSTRIAL APPLICABILITY

According to the present invention, the method for improving the stability of the recombinant plasmid vector comprising the *vapk* gene encoding an alkaline protease can be effectively used in the production of the alkaline protease using a *V. metschnikovii* strains.

**Claims**

1.    A recombinant plasmid vector pSP1 in which the *par* gene is cloned into a recombinant plasmid vector pSBCm (KCCM-10142) comprising the *vapk* gene encoding an alkaline protease to improve the stability of plasmid vector itself.

2.    A method for producing the recombinant plasmid vector pSP1 of claim 1, by inserting the *par* gene into between *Pvu*II and *Hin*cII recognition sites of a recombinant plasmid vector pSBCm (KCCM-10142) comprising the *vapk* gene encoding an alkaline protease derived from *Vibrio* sp., wherein the *par* gene has the function of improving the stability of the plasmid vector itself.

3.    A transformed host cell comprising the recombinant plasmid vector pSP1 of claim 1.

4.    The transformed host cell according to claim 3, wherein said host cell comprises *Escherichia coli* or *Vibrio metschnikovii.*

5.    A method for producing an alkaline protease, VapK, using the transformed host cell of claim 3 or 4.

**Patentansprüche**

1.    Ein rekombinanter Plasmidvektor pSP1 in welchen das *par* Gen in einen rekombinanten Plasmidvektor pSBCm (KCCM-10142) kloniert ist, umfassend das *vapk* Gen, welches für eine Alkalische Protease kodiert um die Stabilität des Plasmidvektors selbst zu verbessern.

**2.** Ein Verfahren zur Herstellung des rekombinanten Plasmidvektors pSP1 aus Anspruch 1, durch Einsetzen des *par* Gens zwischen die Erkennungsstellen *Pvu*II und *Hinc*II eines rekombinanten Plasmidvektors pSBCm (KCCM-10142), umfassend das *vapk* Gen, welches für eine Alkalische Protease, abstammend aus *Vibrio* sp., kodiert, wobei das *par* Gen die Funktion hat, die Stabilität des Plasmidvektors selbst zu verbessern.

**3.** Eine transformierte Wirtszelle, umfassend den rekombinanten Plasmidvektor pSP1 aus Anspruch 1.

**4.** Die transformierte Wirtszelle gemäß Anspruch 3, wobei genannte Wirtszelle *Escherichia coli* oder *Vibrio metschnikovii* umfasst.

**5.** Ein Verfahren zur Herstellung einer Alkalischen Protease, VapK, unter Benutzung der transformierten Wirtszelle aus Anspruch 3 oder 4.

**Revendications**

**1.** Vecteur plasmidique recombinant pSP1, dans lequel le gène *par* est cloné dans un vecteur plasmidique recombinant pSBCm (KCCM-10142) comprenant le gène *vapk* codant pour une protéase alcaline afin d'améliorer la stabilité du vecteur plasmidique lui-même.

**2.** Procédé de production du vecteur plasmidique recombinant pSP1 selon la revendication 1, en insérant le gène *par* entre les sites de reconnaissance *Pvu*II et *Hinc*II d'un vecteur plasmidique recombinant pSBCm (KCCM-10142) comprenant le gène *vapk* codant pour une protéase alcaline dérivé de *Vibrio* sp., dans lequel le gène *par* a pour fonction d'améliorer la stabilité du vecteur plasmidique lui-même.

**3.** Cellule hôte transformée comprenant le vecteur plasmidique recombinant pSP 1 selon la revendication 1.

**4.** Cellule hôte transformée selon la revendication 3, dans laquelle ladite cellule hôte comprend *Escherichia coli* ou *Vibrio metschnikovii.*

**5.** Procédé de production d'une protéase alcaline, VapK, en utilisant la cellule hôte transformée selon la revendication 3 or 4.

FIG. 1

**FIG. 2**

**FIG. 3**

**EP 1 303 626 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 199912588